# EUROPEAN PATENT APPLICATION

(11) **EP 2 977 450 A1**
(43) Date of publication of application: **27.01.2016**
(21) Application number: 14769975.5
(22) Date of filing: 19.03.2014
(51) Int. Cl.: C12N 5/10, A61K 35/12, C12N 5/09, C12N 5/095

(54) **METHOD FOR OBTAINING CELL MASS CONTAINING CANCER STEM CELL**

(30) Priority: 19.03.2013 US 201361803314 P
(71) Applicant: National University Corporation Okayama University, Okayama-shi, Okayama 700-8530 (JP); LSIP, LLC, Chiyoda-ku Tokyo 100-0005 (JP)
(72) Inventor: SENO, Masaharu, Okayama-shi Okayama 700-8530 (JP); MIZUTANI, Akifumi, Okayama-shi Okayama 700-8530 (JP); KASAI, Tomonari, Okayama-shi Okayama 700-8530 (JP)
(74) Representative: Cabinet Plasseraud
(86) International application number: PCT/JP2014/057572
(87) International publication number: WO 2014/148562

(57) **Abstract**

The present invention relates to a method of obtaining a cell population containing cancer stem cells, which comprises culturing iPS cells in the presence of culture supernatant of cancer cells.

## Description

### TECHNICAL FIELD

The present invention relates to a method of obtaining a cell population containing cancer stem cells.

### BACKGROUND ART

Cancer is believed to be a genetic disease and develops as a result of the accumulation of the effects of a series of mutations occurring in basically four or more genes over time, as proposed by the "theory of multistage carcinogenesis." Depending on the organ affected or differences in the cell surface protein specifically expressed, there are numerous types of cancer that has developed; however, each cancer is considered to be a homogeneous tissue composed of a single type of cancer cell. In light of this, each anti-cancer agent, particularly one called as a molecular targeted anti-cancer agent, is designed to attack a single or limited types of cancer cells by targeting a "molecular target" such as a protein specifically expressed. In general, therapeutic drugs and methods are decided by determining and diagnosing the specificity of cancer based on the proteins expressed on the cancer cell surface or the biomarkers such as "tumor markers", which are released from cancer cells and present in body fluids, for example.

As a reason for refractory nature of cancer, the involvement of cancer stem cells is proposed (the cancer stem cell theory). Although isolation of cancer stem cells from various types of cancer (patient tissues) has been reported, isolation and purification of cancer stem cells are difficult due to their low abundance and difficulties in stable maintenance in culture, and this has been impeding large-scale research and analysis. Moreover, not all cancer stem cells have been isolated. It is assumed that the type of cancer cell in a tumor tissue varies from patient to patient and that it is heterogeneous and limited. In view of this, and combined with difficulties in grasping all kinds of cancer cells, many researchers express negative opinions on the existence of cancer stem cell per se or the role played by cancer stem cells in the progression and aggravation of cancer. Japanese Patent Laid-Open No. 2010-13380 (incorporated herein by reference) discloses a method of screening a substance which kills cancer stem cells by using cancer stem cells.

Conventional cancer therapies using anti-cancer agents are always beset with problems such as recurrence and metastasis of cancer. The therapeutic effect is expressed in terms of, for example, the "five-year survival rate," and the overall survival (OS) or survival benefit is used as the end point (index). That is, practically, a common perception is that the "radical cure" of cancer is difficult. It is speculated that the "radical cure" of cancer is rarely achieved due to the following reason. That is, in the end, anti-cancer agents, including molecular targeted drugs, are effective only on specific cancer cells in a population, and not on the remaining cancer cells exhibiting heterogeneity or cancer stem cells. As a result, the therapeutic operation incidentally ends up in assisting the proliferation of those remaining cancer cells and cancer stem cells, leading to recurrence and metastasis. It is thus presumed that there are few cases where cancer is "radically cured." What is demanded is not only a search for new therapeutic methods and therapeutic targets, but also an understanding of and the measure against the "heterogeneity" of cancer cells.

Cancer stem cells can be concentrated using a marker protein such as CD133 and CD44 as an index by, for example, an isolation method using magnetic beads (Jin et al. 2008. Neuroscience; 154, 541 to 550 (incorporated herein by reference), Diamond CD133 Isolation Kit manufactured by Miltenyi Biotec GmbH), a method involving separation and collection by flow cytometry (Griguer et al. 2008. PlosOne; 3 (11) e3655 (incorporated herein by reference)), and laser microdissection (LMD6500 & LMD7000 manufactured by Leica Camera AG). For culture and maintenance of isolated or separated cancer stem cells, a gel polymerized with hyaluronic acid or a gel obtained by polymerization between hyaluronic acid and gelatin (Rehfeldt et al. 2012 Integr. Biol.; 4, 422 to 430 (incorporated herein by reference), Hystem manufactured by Sigma-Aldrich Corporation), a collagen gel (Matrigel manufactured by Nippon Becton Dickinson Company, Ltd.) and the like can be used. Conventional technology for isolating CD44-expressing cells has required expensive instruments and reagents, a space for installing the instruments to be used and relevant specialized skills to use the instruments. In addition, as separated cells had to be maintained in a polymerized gel or on the surface of a gel, it was extremely difficult to observe cells in the bright field when the cells does not expresses fluorescent proteins, or staining or the like of the cells is impossible. Further, preparation of a uniform gel and maintenance of the cell condition at a constant level were difficult. Also, collection and passage of cultured cells and collection of factors secreted by the cells or the like were extremely difficult. In addition, another problem was that exposure of the cells to drugs, growth factors, cytokines, and the like was difficult.

### CITATION LIST

### PATENT DOCUMENT

Patent Document 1: JP2010-13380 A

### NON PATENT DOCUMENT

Non Patent Document 1: Jin et al. 2008. Neuroscience; 154,541-550.
Non Patent Document 2: Griguer et al. 2008. PlosOne; 3(11)e3655.
Non Patent Document 3: Rehfeldt et al. 2012 Integr.Biol.; 4,422-430.

### SUMMARY OF THE INVENTION

An object of the present invention is to provide a method of obtaining a cell population containing cancer stem cells (particularly, a cell population containing cancer cells). Another object of the present invention is to provide a method of concentrating CD44-expressing cells.

The present invention is directed to induce, for the first time, cancer stem cells by culturing iPS cells. Although the induced cancer stem cells may be contaminated with cancer cells that have differentiated from the cancer stem cells, the cancer stem cells can be used after removing the contaminating cancer cells. Also, it is known that many cancer stem cells express a high level of CD44. It was found that when hyaluronic acid was added to the medium to concentrate cancer stem cells, cells expressing a high level of CD44 were unexpectedly efficiently concentrated, whereby the present invention was completed. Although specific examples will be shown below, the present invention is not limited thereto.
1. A method of obtaining a cell population containing cancer stem cells, which comprises culturing iPS cells in the presence of culture supernatant of cancer cells.
2. The method of the aforementioned 1, wherein the iPS cells are human cells.
3. The method of the aforementioned 1 or 2, wherein the period of culture is 2 weeks to 2 months.
4. The method of any one of the aforementioned 1 to 3, wherein the cell population obtained by the method contains cancer cells.
5. A cell population obtained by the method of any one of the aforementioned 1 to 4.
6. A method of obtaining cancer stem cells, which comprises isolating cancer stem cells contained in the cell population of the aforementioned 5.
7. Cancer stem cells obtained by the method of the aforementioned 6.
8. A method of screening an anti-cancer agent, which comprises the steps of:
   (1) bringing the cell population containing cancer stem cells of the aforementioned 5 or the cancer stem cells of the aforementioned 7 into contact with a candidate of an anti-cancer agent, and
   (2) determining the effect of the candidate on the cell population or the cells.
9. The method of the aforementioned 8, further comprising the step of selecting a candidate which inhibits proliferation of the cell population or the cells.
10. The method of the aforementioned 8 or 9, which comprises the step of bringing the cell population containing cancer stem cells of the aforementioned 5 into contact with a candidate of an anti-cancer agent.
11. A method of concentrating CD44-expressing cells, which comprises culturing a cell population containing CD44-expressing cells in the presence of hyaluronic acid under the non-adherent condition.
12. The method of the aforementioned 11, wherein the cell population is a human cell population.
13. The method of the aforementioned 11 or 12, wherein the period of the culture is 2 weeks to 2 months.
14. A cell population containing CD44-expressing cells concentrated by the method of any one of the aforementioned 11 to 13.

A cell population containing various cancer stem cells that constitute tumor in the living body and various cancer stem cells can be obtained by the present invention.

### BRIEF DESCRIPTION OF THE DRAWINGS

FIG. 1 illustrates the formation of tumor by miPS-LLCcm cells;
FIG. 2 illustrates the histological evaluation of tumor formed by miPS-LLCcm cells;
FIG. 3 illustrates the results of immunostaining of tumor formed by miPS-LLCcm cells;
FIG. 4 illustrates the cells forming vascular-like structure in Matrigel (R);
FIG. 5 illustrates a primary cell culture isolated from malignant tumor derived from miPS-LLCcm cells and a suspension culture thereof. The primary culture shows the cells obtained from tumor mass formed in a nude mouse into which miPS-LLCcm cells were transplanted. The suspension culture shows spheroids obtained by culturing miPS-LLCcm cells in the serum-free medium. The pictures on the left show GFP expression;
FIG. 6 illustrates the results of immunostaining of tumor formed from the spheroids of miPS-LLCcm cells;
FIG. 7 Left) illustrates the expression of p53, which is a representative cancer suppressor gene, and Right) illustrates the expression of matrix metalloproteinase 2 (MMP2), which is involved in the infiltration and metastasis of cancer;
FIG. 8 illustrates the expression of stem cell marker genes;

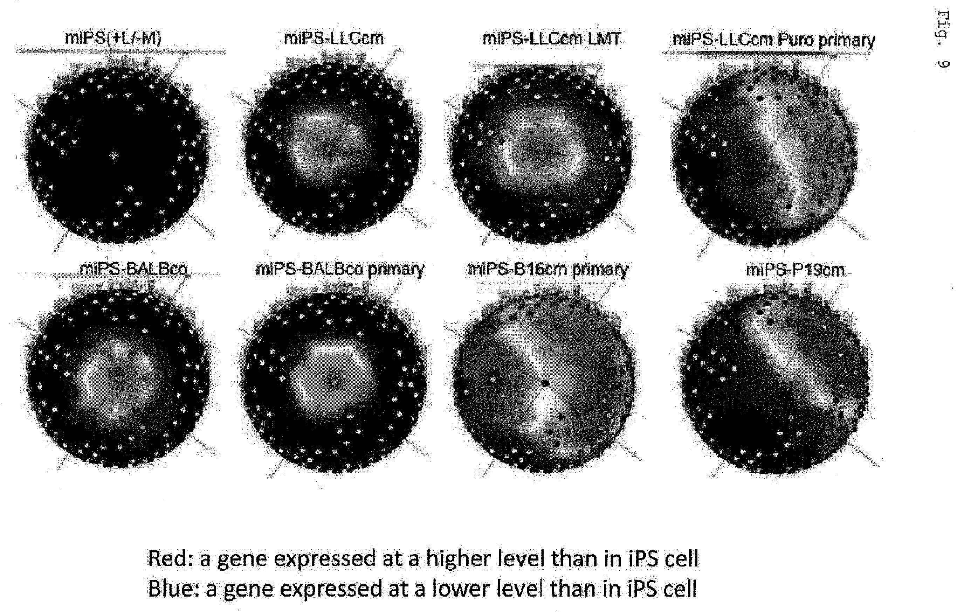

FIG. 9 illustrates the visualization of the gene expression profile of mouse iPS-CSC by spherical self-organizing mapping. In the figure, in the second and third cells from the left in the top row and in the leftmost and second cells from the left in the bottom row, mainly, a red color (a gene expressed at a higher level than in iPS cells) is shown in the center, which is surrounded by a blue color (a gene expressed at a lower level than in iPS cells). In the rightmost cells in the top row and in the third cells from the left and the rightmost cells in the bottom row, mainly, a red color is shown in the right area and a blue color is shown in the left area;
FIG. 10 illustrates the comparison of gene expression among individual cells;
FIG. 11-1 illustrates human iPS cells (1) cultured in the presence of the culture supernatant of cancer cells;
FIG. 11-2 illustrates human iPS cells (2) cultured in the presence of the culture supernatant of cancer cells;
FIG. 11-3 illustrates human iPS cells (3) cultured in the presence of the culture supernatant of cancer cells;
FIG. 11-4 illustrates human iPS cells (4) cultured in the presence of the culture supernatant of cancer cells;
FIG. 11-5 illustrates human iPS cells (5) cultured in the presence of the culture supernatant of cancer cells;
FIG. 12 illustrates the induction of cancer stem cells from human iPS cells;
FIG. 13 illustrates the detection of podocalyxin with rBC2LCN-FITC;
FIG. 14 is a set of pictures showing the case without the addition of rBC2LCN-FITC;
FIG. 15 illustrates the tumor formation in a nude mouse;
FIG. 16 illustrates the induction of stem cells from OCC-hiPS-29 cells;
FIG. 17 illustrates the self-renewal ability of OCC-hiPS-29 cells;
FIG. 18 illustrates the visualization of the gene expression profile of human iPS-CSC by spherical self-organizing mapping. In the figure, a blue color (a gene expressed at a lower level than in iPS cells) is shown in the black area on the left side, while a red color (a gene expressed at a higher level than in iPS cells) is shown in the lower right and left corner areas (in the fourth cells from the left and the rightmost cells in the bottom row) or in the lower right corner area (in all the other cells).
FIG. 19 schematically illustrates an experiment using human glioma-derived U251MG cells.
FIG. 20 illustrates culture in the medium with hyaluronic acid (HA);
FIG. 21 illustrates a study of the HA concentration;
FIG. 22 illustrates culture in non-adherent dishes. With: medium with HA, Without: medium without HA (normal medium);
FIG. 23 illustrates dissociation and self-renewal of cells from spheres;
FIG. 24-1 illustrates the comparison of the CD44 gene expression level. A: A172 cells;
FIG. 24-2 illustrates the comparison of the CD44 gene expression level. B: U251MG cells;
FIG. 24-3 illustrates the comparison of the CD44 gene expression level. C: U251MG-P1 cells;
FIG. 25 illustrates the expression level of the CD44 gene in U251MG-P1 cells relative to U251MG cells;
FIG. 26 illustrates the tumor formation by U251MG cells cultured in the medium with HA;
FIG. 27 illustrates the comparison of the CD44 protein expression level. A: Adherent dishes with normal medium; N: Non-adherent dishes with normal medium; and H: Non-adherent dishes with the medium with HA;
FIG. 28 illustrates the detection of the astrocyte marker protein (GFAP); and
FIG. 29 illustrates culture of SkOv-3 cells in the medium with HA.

### DETAILED DESCRIPTION OF THE EMBODIMENTS

### 1. Method of obtaining a cell population containing cancer stem cells

iPS cells are cells derived from a vertebrate, preferably a mammal. Examples of the mammal include human, mouse, rat, hamster, rabbit, dog, cat, cow, horse, sheep, and monkey. The iPS cells are preferably derived from primates or rodents, more preferably derived from human or mouse, and even more preferably derived from human. As the iPS cells, for example, iPS-MEF-Ng-20D17, iPS-MEF-Ng-178B-5, iPS-MEF-Fb/Ng-440A-3, iPS-MEF-Ng-492B-4, iPS-Stm-FB/gfp-99-1, iPS-Stm-FB/gfp-99-3, iPS-Hep-FB/Ng/gfp-103C-1, iPS-L1, iPS-S1, 253G1, 409B2, 454E2, HiPS-RIKEN-1A, HiPS-RIKEN-2A, HiPS-RIKEN-12A, Nips-B2, and the like may be used.

Cancer cells are cells derived from a vertebrate, preferably a mammal. Examples of the mammal include human, mouse, rat, hamster, rabbit, dog, cat, cow, horse, sheep, and monkey. The cancer cells are preferably derived from primates or rodents, more preferably derived from human or mouse, and even more preferably derived from human. Examples of the cancer cells include cells of lung cancer, embryonal carcinoma, malignant melanoma, breast cancer, colorectal cancer, pancreatic cancer, brain tumor, liver cancer, hepatoma, gastrointestinal tract cancer, ovarian cancer, leukemia, and lymphoma. As the cancer cells, for example, cells such as LLC, P19-CL6, B16-F10, BALB MC.E12, A172, U251-MG, MDA-MB-415, MDA-MB-231, T-47D, ZR-75-1, SK-BR-3, BT-549, MCF7, HT-29, PANC1, SKOV3, HepG2, Huh-7, PLC/PRF-5, ECC, CW-2, PMF-ko14, MY, MOLT4, KLM-1, PK8, PK59, A549, Li-7, OVCAR-3, Lu99B, RERF-LC-KJ, OVK18, and RERF-LC-AI may be used. In one aspect, the cancer cell is selected from LLC, P19-CL6, B16-F10, BALB MC.E12, A172, MDA-MB-415, T-47D, BT-549, HT-29, SKOV3, MY, MOLT4, PK59, A549, Li-7, OVCAR-3, Lu99B, and RERF-LC-KJ. In one aspect, the cancer cell is selected from A172, MDA-MB-415, T-47D, BT-549, HT-29, SKOV3, MY, MOLT4, PK59, A549, Li-7, OVCAR-3, Lu99B, and RERF-LC-KJ.

The iPS cells and the cancer cells that are used to obtain culture supernatant may or may not be derived from the same origin. For example, iPS cells derived from human may be cultured in the presence of the culture supernatant of cancer cells derived from mouse, rat, or monkey. In one aspect, the iPS cells and the cancer cells that are used to obtain culture supernatant are derived from the same origin.

As the medium to obtain the culture supernatant of cancer cells, any cell culture medium can be selected and used according to the cancer cells to be used. Examples thereof include, but are not limited to, DMEM, Glasgow's MEM (GMEM), EMEM, MEMα, RPMI-1640, Ham's F-10, Medium 199, Ames' Medium, BGJb Medium, EHAA, CMRL-1066 Medium, Fischer's Medium, IMDM, Leibovitz, McCoy's 5A Modified Medium, NCTC Medium, Swim's S-77 Medium, Waymouth Medium, William's Medium E, Ham's F-12, and MCDB medium. The culture supernatant of cancer cells is obtained by, for example, washing cancer cells that have reached 80% confluency in an incubator with fresh medium, and then culturing the cells in fresh medium further for 24 hours to 72 hours, preferably for 24 hours to 48 hours. The medium to obtain the culture supernatant may or may not contain serum. The culture supernatant may be used after filteration through a 0.45 µm-pore or 0.22 µm-pore filter. Since a cancer stem cell-inducing factor is considered to be contained in a liquid fraction of the culture supernatant of cancer cells, the liquid fraction of the culture supernatant of cancer cells obtained by removing macromolecules such as cell fragments and exosomes from the culture supernatant may be used as the "culture supernatant of cancer cells" in the method of the present invention.

As the medium for culturing iPS cells, any stem cell culture medium can be used, and examples thereof include, but are not limited to, DMEM, Repro FF2 or Repro stem (ReproCELL Inc.), and the DMEM-F12 medium containing non-essential amino acid, 200 mM L-glutamine, KSR, and 0.1 M 2-mercaptoethanol/PBS.

The iPS cells are cultured for approximately two weeks to two months, preferably for approximately four weeks to two months in the presence of the culture supernatant of cancer cells. For example, a part (for example, half) or all of the iPS cell medium is exchanged for the culture supernatant of cancer cells once every one to four days. The presence of cancer stem cells in the cell population obtained can be confirmed based on the ability to form malignant tumor in a nude mouse, specifically by the pathological diagnosis of specimens (for the presence or absence of active cell division, atypical cells, angiogenesis, and the like). The presence of cancer stem cells in the cell population obtained can also be confirmed by, for example, evaluation of the ability to form vascular structure in vitro, variation in the gene expression of cancer-suppressing factors, as represented by p53, and expression of a known cancer stem cell marker such as CD44.

Cancer stem cells are cells possessing differentiation potency, which ultimately becomes terminally differentiated cancer cells. Cancer stem cells are positioned between iPS cells and cancer cells, and there are various cancer stem cells in different stages of differentiation. The cell population obtained by the method of the present invention is a heterogeneous cell population containing various cancer stem cells, and it may also contain cancer cells. The cell population obtained by the method of the present invention can be separated into individual cancer stem cells in different stages of differentiation by a cell sorter or the like, and it can also be separated into single cells in accordance with publicly known technique. Also, given that cancer stem cells are considered to form spheroids by self-renewal of a single cell, it is also possible to perform single cell cloning by spheroid culture, and then separating spheroids into single cells.

According to the present invention, a cell population containing various cancer stem cells that constitute tumor in the living body can be prepared in vitro from pluripotent iPS cells. As the cell population obtained by the present invention is induced from iPS cells in a sufficiently short time, gene mutation is not considered to be introduced. That is, according to the present invention, a variety of kinds of cancer stem cells free from accumulation of gene mutations can be comprehensively induced. Using the cell population and the cancer stem cells obtained by the present invention, cells and cancer stem cells emerging in the living body can be comprehensively and systemically analyzed and studied. For example, research findings obtained by comprehensive gene expression analysis by, for example, a next generation sequencer, epigenetic analysis, proteome analysis, and metabolome analysis can be compiled into a database as cell information and utilized for the development of anti-cancer agents.

The cell population or cancer stem cells obtained by the method of the present invention can be used in a method of screening an anti-cancer agent. In order to bring the cell population or the cancer stem cells obtained by the method of the present invention into contact with a candidate of an anti-cancer agent, the candidate of an anti-cancer agent may be added to the medium of the cell population or the cancer stem cells obtained by the method of the present invention.

Examples of the candidate include organic low molecular weight compounds, nucleic acids, sugars, lipids, amino acids, proteins, antibodies, compound libraries prepared by combinatorial chemistry technology, random peptide libraries prepared by solid-phase synthesis or a phage display method, and natural components (for example, components derived from microorganisms, animals, plants, or marine organisms). The concentration of the candidate in the medium may be approximately 1 ppb to 1%, preferably approximately 1 ppm to 0.1%. When the candidate has poor water solubility, it can be dissolved in an appropriate organic solvent such as ethanol and DMSO, or dissolved in the medium in the form of a salt of the candidate with a nontoxic acid or base.

The effect of the candidate can be determined based on the cell proliferation, for example. A candidate inhibiting the cell proliferation of the cell population containing cancer stem cells or the cancer stem cells obtained by the method of the present invention can be selected as a substance useful as an anti-cancer agent. The cell proliferation may be determined by counting the number of viable cells under a microscope, or by the MTT assay, the trypan blue exclusion assay, the intracellular ATP measurement method, the metabolic activity assay using resazurin (QBlue assay), or the AlamarBlue assay. For example, a candidate inhibiting the cell proliferation by 10% or more, preferably 20% or more, more preferably 50% or more than does a control is selected as a substance useful as an anti-cancer agent.

### 2. Method of concentrating CD44-expressing cells

CD44 is a type I transmembrane glycoprotein receptor for hyaluronic acid. It is specifically highly expressed on the surface of cancer cells such as glioma, and is known to be a cancer stem cell marker of human pancreatic cancer, prostate cancer, breast cancer, and the like. The functions of CD44 include, for example, cell aggregation, maintenance of the pericellular matrix, matrix-cell signaling, and control of orientation of matrix metalloproteinase (MMP) (cell migration and infiltration).

The cell population containing CD44-expressing cells is a cell population derived from a vertebrate, preferably a mammal. Examples of the mammal include human, mouse, rat, hamster, rabbit, dog, cat, cow, horse, sheep, and monkey. The cell population containing CD44-expressing cells is preferably derived from primates or rodents, more preferably derived from human or mouse, and even more preferably derived from human. It is preferable that the cell population containing CD44-expressing cells contain cells expressing CD44 at such a high level that CD44 is detectable at the protein level by Western blotting, immunostaining, or the like. In one aspect, CD44-expressing cells are cancer stem cells. As the cell population containing CD44-expressing cells, for example, U251MG cells and A172 cells (human glioma), SkOv-3 cells (human ovarian cancer), 4T1 cells (mouse breast cancer), MDA-MB-231 cells (human breast cancer), AsPC-1 cells (human pancreatic cancer), and PC-3 cells (human prostate cancer) may be used.

Hyaluronic acid is composed of a repeating unit of disaccharide consisting of N-acetylglucosamine and glucuronic acid. The molecular weight of hyaluronic acid used in the present invention is approximately 500 to 2,000,000, preferably approximately 1,000 to 1,500,000, more preferably approximately 3,000 to 1,000,000. According to the method of the present invention, naturally-occurring hyaluronic acid may be used as it is, and also a low-molecular weight hyaluronic acid having a reduced molecular weight obtained by degradation with an enzyme (hyaluronidase) or hydrolysis with acid may be used.

In the present specification, the term "hyaluronic acid" encompasses both a high-molecular weight hyaluronic acid and a low-molecular weight hyaluronic acid. A "high-molecular weight hyaluronic acid" refers to hyaluronic acid having a molecular weight of 100,000 or more, preferably 300,000 or more, more preferably 500,000 or more, even more preferably 700,000 or more, and particularly 1,000,000 or more. Naturally-occurring hyaluronic acid falls under the "hyaluronic acid" having a great molecular weight (i.e., a high-molecular weight hyaluronic acid) mentioned above.

The "low-molecular weight hyaluronic acid" used in the present invention can be obtained by chemically treating hyaluronic acid with an enzyme, an acid, an alkali or the like to reduce the molecular weight of the hyaluronic acid, or by separating hyaluronic acid having a molecular weight equal to or less than a predetermined value from naturally-occurring hyaluronic acid. The "low-molecular weight hyaluronic acid" refers to hyaluronic acid having a molecular weight of 100,000 or less, and the upper limit of the molecular weight is approximately 100,000, 70,000, 50,000, 30,000, 10,000, or 5,000, while the lower limit of the molecular weight is approximately 500, 1,000, 2,000, and 3,000.

Hyaluronic acid may be derived from an animal, for example, derived from chicken combs and umbilical cord. Hyaluronic acid may also be derived from microorganisms such as lactic acid bacteria and streptococci.

Hyaluronic acid is added to the medium at a concentration of approximately 0.1 µg/ml to 5000 µg/ml, preferably approximately 1 µg/ml to 3000 µg/ml, more preferably approximately 5 µg/ml to 1000 µg/ml, and even more preferably approximately 10 µg/ml to 200 µg/ml.

The cell population containing CD44-expressing cells may be cultured in a conventional culture method and a culture condition according to each cell type. Hyaluronic acid may be added to any cell culture medium, and examples of the cell culture medium include, but are not limited to, DMEM, Glasgow's MEM (GMEM), EMEM, MEMα, RPMI-1640, Ham's F-12, and MCDB. Further, an antibiotic such as penicillin and streptomycin, serum, various growth factors or differentiation inducing factors, and the like may be added to the medium.

A culture container may be any culture container or culture dish suitable for non-adherent culture such as flasks, schales petri dishes, plates, tissue culture tubes, trays, culture bags, roller bottles, or hollow fibers. In one embodiment, the culture container is a culture container having a non-cell-adherent inner bottom surface. Cultured cells may adhere to the non-cell-adherent inner bottom surface so long as the formation of spheroids (spheres) is not inhibited, or cultured cells may not adhere to the non-cell-adherent inner bottom surface at all.

A culture container having a non-cell-adherent inner bottom surface may be any publicly known or commercially available non-cell-adherent culture container, and examples thereof include, but are not limited to, a culture container produced by injection-molding general-purpose plastic such as polystyrene without applying special surface treatment, a culture container having a scaffold made of non-woven cloth or porous film, a molded plastic culture container having a finely corrugated surface, a culture container made of a highly hydrophilic substance such as polyethylene glycol, polyhydroxyethylmethacrylate, and an ethylene vinyl alcohol copolymer, a culture container with its surface coated with a substance capable of adding hydrophilicity to the surface performance such as a surfactant and phospholipid, and a culture container acquiring hydrophilicity by surface treatment such as plasma treatment.

The number of cells to be cultured is appropriately determined in consideration of capacity of the culture container, the cell type, the final size of spheroid, and the like. For example, the cells are seeded in a culture container at a final concentration of 10⁴ cells to 10⁷ cells, preferably 10⁴ cells to 10⁶ cells per mL medium. The cells may be cultured either by shaking culture or static culture. The culture solution can be exchanged without limitation as long as spheroids are formed. The culture solution may be exchanged once or twice or more per day or once every two to four days. Also, the culture solution may be exchanged after precipitation of the cells by centrifugation. The culture period is approximately two to 20 days, preferably approximately two to 14 days, more preferably approximately five to 14 days. The culture temperature is preferably around 37°C.

By culturing a cell population containing CD44-expressing cells in the presence of hyaluronic acid under non-adherent culture conditions, spheroids of CD44-expressing cells are formed, whereby CD44-expressing cells are concentrated. The CD44-expressing cells that have formed spheroids by the method of the present invention express a higher level of CD44. Further, cell proliferation of the CD44-expressing cells is promoted and their cellular functions are enhanced. The concentrated CD44-expressing cells can be isolated and purified by a publicly known method.

As to the size of spheroid, the diameter of the spheroid is approximately 10 to 500 µm, preferably 50 to 200 µm, in general. When a spheroid is overgrown, nutrients cannot readily reach the inner part of the spheroid. Therefore, it is desirable to separate a spheroid once it reaches an appropriate size. Spheroids can be repeatedly formed when an obtained spheroid is dissociated into cells, and then the dissociated cell is cultured again in non-adherent culture. CD44-expressing cells can be concentrated through the formation of spheroids, and CD44-expressing cells having a higher purity (homogeneous character) can be obtained by the repeated spheroid formation.

The present invention will be described in more detail based on Examples hereinafter.

### Examples

### Example 1. Induction of cancer stem cells from mouse iPS cells

### (1) Culture of mouse iPS cells

Mouse iPS cells (iPS-MEF-Ng-20D17, purchased from Cell Bank, RIKEN BioResource Center) were cultured and maintained in the DMEM medium (containing 15% FCS, 0.1 mM NEAA, 2 mM L-glutamine, 0.1 mM 2-mercaptoethanol, 1000 U/ml LIF, 50 U/ml penicillin, and 50 U/ml streptomycin) at 37°C in 5% CO₂ on MEF cells. The mouse iPS cells used in the present experiment stably express the GFP gene in the undifferentiated state, but once differentiated, they stop expressing the gene (Okita, K. et al. Nature 448: 313 to 317 (2007), incorporated herein by reference).

### (2) Preparation of the culture supernatant of cancer cells (conditioned medium)

The mouse Lewis lung carcinoma cell line LLC cells were cultured in the DMEM medium containing 10% serum. The mouse embryonal carcinoma cells, P19-CL6 cells, were cultured in the αMEM medium containing 10% serum (containing 10% FCS, 50 U/ml penicillin, and 50 U/ml streptomycin). The mouse malignant melanoma cells, B16-F10 cells, and breast cancer cells, BALB MC.E12 cells, were cultured in the MEM medium containing 10% serum (containing 10% FCS, 50 U/ml penicillin, and 50 U/ml streptomycin) at 37°C in 5% CO₂. After culturing each of these cells to confluency, the culture supernatant was collected and filtered through a 0.45 µm filter, whereby a conditioned medium was obtained.

### (3) Induction of cancer stem cells (conditioned medium)

In gelatin-coated dishes having a diameter of 60 mm, 7 × 10⁵ mouse iPS cells were freshly seeded and cultured with the DMEM medium (containing 15% FCS, 0.1 mM NEAA, 2 mM L-glutamine, 0.1 mM 2-mercaptoethanol, 50 U/ml penicillin, and 50 U/ml streptomycin). From the day after the cells were seeded, half of the medium was exchanged every day for the conditioned medium prepared from various cancer cells, and culture was continued for four weeks. When the proliferation of the mouse iPS cells was confirmed, the cells were collected by trypsin treatment and 7 × 10⁵ cells were freshly seeded in new gelatin-coated dishes having a diameter of 60 mm with the DMEM medium (containing 15% FCS, 0.1 mM NEAA, 2 mM L-glutamine, 0.1 mM 2-mercaptoethanol, 50 U/ml penicillin, and 50 U/ml streptomycin) and eight hours later an equal amount of conditioned medium was added to the medium. The culture of the cells was continued for four weeks. The induced cells were maintained in the DMEM medium (containing 15% FCS, 0.1 mM NEAA, 2 mM L-glutamine, 0.1 mM 2-mercaptoethanol, 50 U/ml penicillin, and 50 U/ml streptomycin).

### (4) Induction of cancer stem cells (co-culture)

Various cancer cells (1.5 × 10⁵ cells) which have been treated with 0.4 µg/ml mitomycin C for induction of proliferation inhibition were seeded in gelatin-coated dishes having a diameter of 60 mm. On the next day, mouse iPS cells (5 × 10⁵ cells) were seeded. When the proliferation of the mouse iPS cells was observed, the cells were passaged. The induction was carried out for four weeks. As a control, iPS cells co-cultured with MEF cells, which were feeder cells, were used.

### (5) Tumorigenicity evaluation

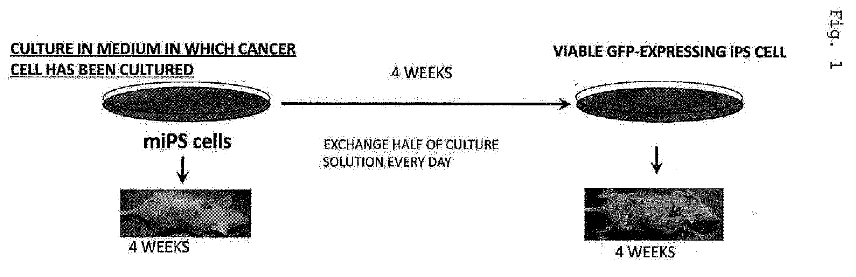

The tumorigenic ability of the cancer stem cells induced from iPS cells was evaluated using nude mice (Balb/c Slc-nu/nu 6 to 8-week-old female) (FIG. 1). In 100 µl of the DMEM medium (containing 10% serum), 4 × 10⁶ miPS-LLCcm cells were suspended and then transplanted in the back of the nude mouse by subcutaneous injection. Four weeks later, tumor formed was excised and fixed in 10% neutral buffered formalin (Wako Pure Chemical Industries, Ltd.) for 24 hours. The tumor was then embedded in paraffin, and sections of 3 µm in thickness were prepared and subjected to hematoxylin/eosin staining. The other cells were also subjected to the same experiment.

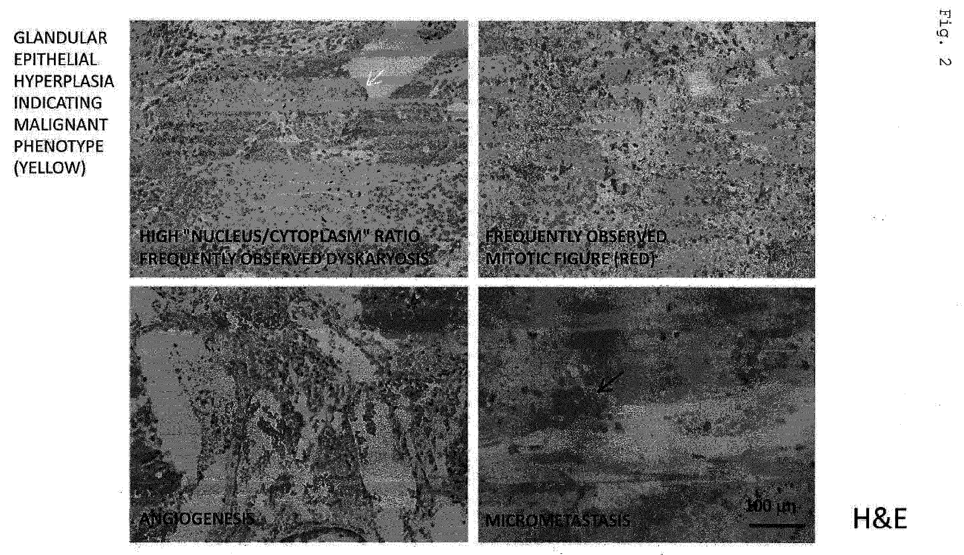

It was confirmed that malignant tumor was formed when a cell population obtained from iPS cells by culture in the presence of the culture supernatant of various cancer cells was transplanted into a nude mouse (Table 1). FIG. 2 illustrates the results of the histological analysis of tumor formed after transplantation of miPS-LLCcm cells. Meanwhile, iPS cells resulted in the formation of teratoma (benign), not malignant tumor.

**[Table 1]**

| Established cell line | Effector cells | Tumor formation | Histological assessment |
|---|---|---|---|
| miPS-feeder+ | MEF | 3/3 | Teratoma (benign) |
| miPS-feeder- | - | 3/3 | Teratoma (benign) |
| miPS-LLCcm* | LLC | 8/8 | Malignant tumor, angiogenesis |
| miPS-P19cm | P19-CL6 | 5/5 | Malignant tumor |
| miPS-B16cm | B16-F10 | 2/3 | Malignant tumor |
| miPS-MC. E12cm | BALB MC. E12 | 3/3 | Malignant tumor |
| miPS-MC. E12c | | 3/3 | Malignant tumor |

| | | | |
|---|---|---|---|
| cm: conditioned medium c: co-culture | | | |

### (6) Immunostaining of tumor sections

The tumor sections were deparaffinized with xylene and heated at 95°C for five minutes in a 10 mM citric acid buffer (pH 6), followed by digestion with protease K (37°C, 30 minutes) for antigen retrieval. The sections were then stained with an anti-GFP antibody (provided by Ayano Sato, Associate Professor at Okayama University, 1:200), an anti-cytokeratin antibody (anti-pan-Cytokeratin (AE1/AE3) antibody, Santa Cruz Biotechnology, Inc., 1:200), and an anti-CD31 antibody (Santa Cruz Biotechnology, Inc., 1:200). Color development was performed using biotinylated secondary antibody (Vector Laboratories), the ABC reagent (Vector Laboratories), and DAB (Vector Laboratories). As counterstaining, hematoxylin staining was carried out.

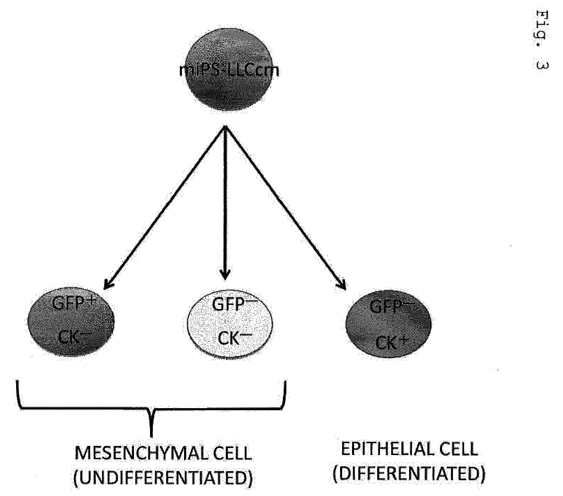

The tumor derived from miPS-LLCcm cells was found to be composed of cells expressing only GFP (GFP+ CK-), cells expressing only cytokeratin (GFP- CK+), and cells expressing neither of them (GFP- CK-) (FIG. 3). GFP+ cells indicate cells remaining undifferentiated, and CK+ cells indicate cells that have differentiated into epithelial cells. A GFP- CK- cells indicate cells that are in the process of differentiation into epithelial cells but have not yet expressed cytokeratin, or cells that have differentiated into cells other than epithelial cells (cells that does not express cytokeratin at least). The above findings indicate that tumor is a heterogeneous population composed of various cells.

### (7) In vitro tube formation assay and immunostaining

In an 8-well chamber slide (Nippon Becton Dickinson Company, Ltd.) coated with Matrigel (R) (Nippon Becton Dickinson Company, Ltd.), 1.4 × 10⁵ miPS-LLCcm cells were seeded and cultured with the EGM2 medium (Takara Bio Inc.) for 24 hours to allow the formation of a vascular tube-like structure. Subsequently, the cells were fixed with 4% paraformaldehyde and then immunostained with anti-CD31 antibody (Santa Cruz Biotechnology, Inc., 1:200) and Texas Red-labeled secondary antibody. The cell nuclei were stained with DAPI and observed under a fluorescence microscope and a confocal microscope.

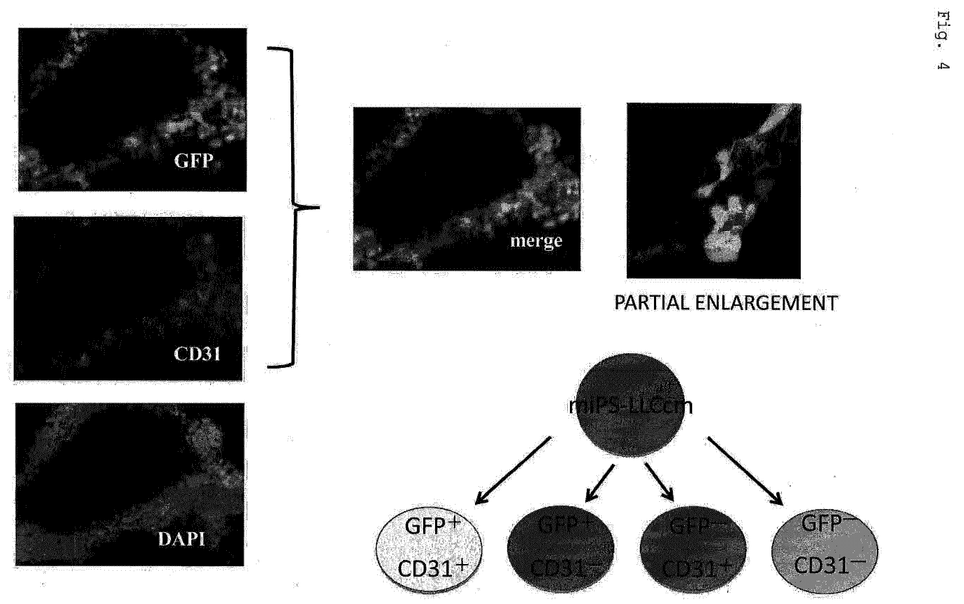

The formation of a vascular-like tube structure by miPS-LLCcm cells was observed. As a result of staining the cells contained in this structure for CD31, which is a vascular endothelial marker, it was found that this structure was composed of GFP positive and CD31 positive (GFP+, CD31+) cells, GFP positive and CD31 negative (GFP+, CD31-) cells, GFP negative and CD31 positive (GFP-, CD31+) cells, and GFP negative and CD31 negative (GFP-, CD31-) cells (FIG. 4). These results indicate that the cell population obtained by the present invention differentiates into cells involved in angiogenesis, and has an ability to form a blood vessel composed of at least four cell types in vitro.

### (8) Evaluation of tumorigenicity of spheroidal cells

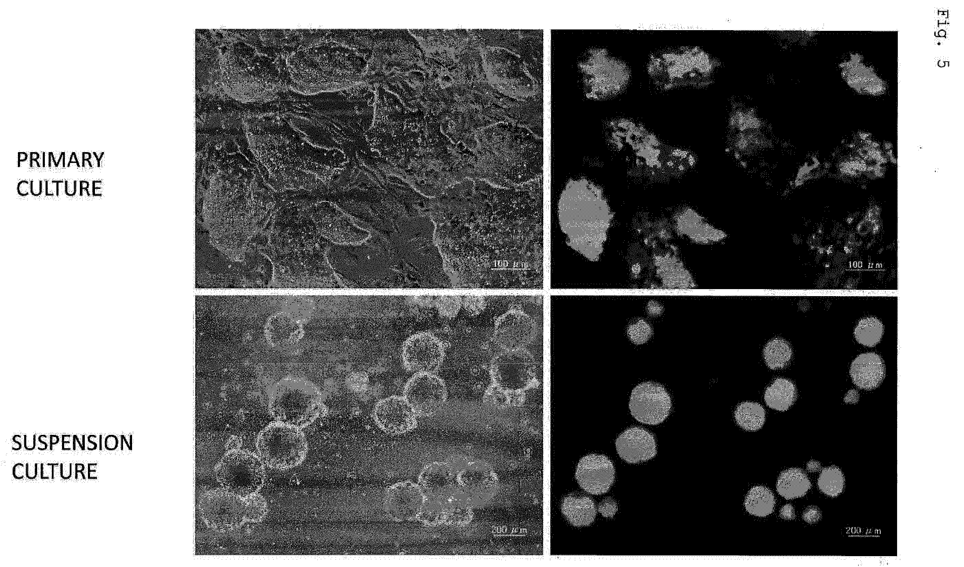

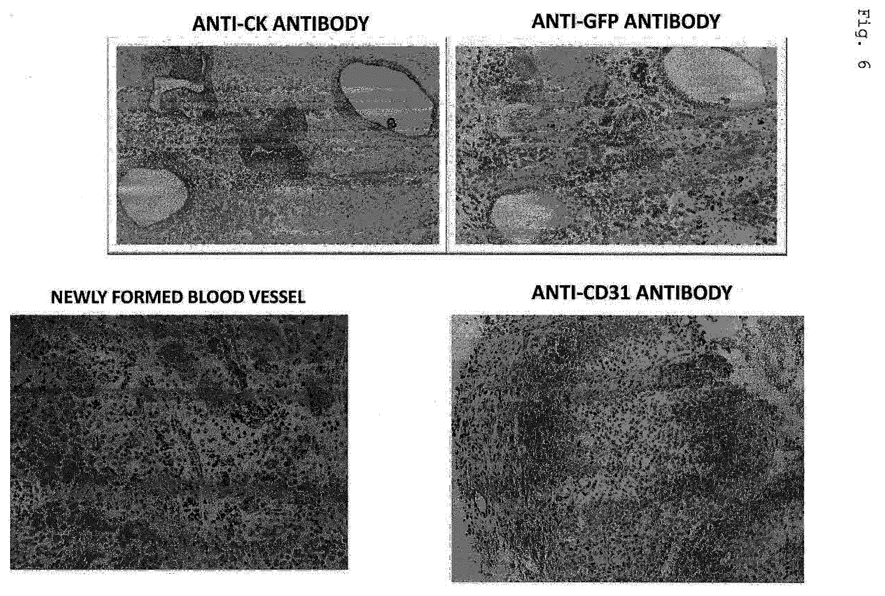

The miPS-LLCcm primary cells were established by primary culture of the tumor formed in a nude mouse by miPS-LLCcm cells in accordance with a publicly known method. The resulting cells contain GFP positive cells as well as GFP negative cells (which were assumed to be differentiated from GFP positive cells). These cells were separated into single cells by trypsin treatment, and then seeded in non-surface-treated dishes at 2 × 10⁴ cells/ml (suspension culture). As the medium, the serum-free DMEM medium (containing 0.1 mM NEAA, 2 mM L-glutamine, 0.1 mM 2-mercaptoethanol, 50 U/ml penicillin, and 50 U/ml streptomycin) was used. The cells were cultured for seven to ten days and the formation of spheroids was observed. The spheroid thus formed was GFP positive. These results indicate that a spheroid is formed by a single type of cell in an anchorage-independent manner, and remains undifferentiated. These findings indicate the self-renewal ability of miPS-LLCcm cells (FIG. 5). As a result of dissociating the spheroid into single cells by trypsin and transplanting 1 × 10⁵ or more cells in a nude mouse, malignant tumor was formed again. In terms of morphology, as in the primary tumor, the resulting malignant tumor was composed of GFP positive cells, CK positive cells, and GFP negative and CK negative cells, and was rich in newly formed blood vessels (FIG. 6). These results indicate that even after repetitive transplantation, miPS-LLCcm cells form morphologically similar tumor.

When the spheroid formation experiment was performed using the cells that were cultured for two weeks with the addition of the conditioned medium of LLC cells and then cultured three weeks in a normal medium, the formation of GFP positive spheroids was observed.

### (9) Lung metastasis of spheroidal cells

In a nude mouse, 1 × 10⁵ cells collected from the spheroid of (8) described above were transplanted through the caudal vein, and four weeks later, tumor formed in the lung was observed. miPS-LLCcm LMT cells were established by primary culture of the tumor formed in accordance with a publicly known method. The cells were maintained in the DMEM medium (containing 15% FCS, 0.1 mM NEAA, 2 mM L-glutamine, 0.1 mM 2-mercaptoethanol, 50 U/ml penicillin, and 50 U/ml streptomycin). Using RNeasy kit (50) (QIAGEN GmbH), RNA was purified from the miPS cells, miPS-LLCcm spheroids, miPS-LLCcm LMT spheroids, and LLC cells, and the expression level was compared between the p53 gene and the MMP2 gene by RT-PCR.

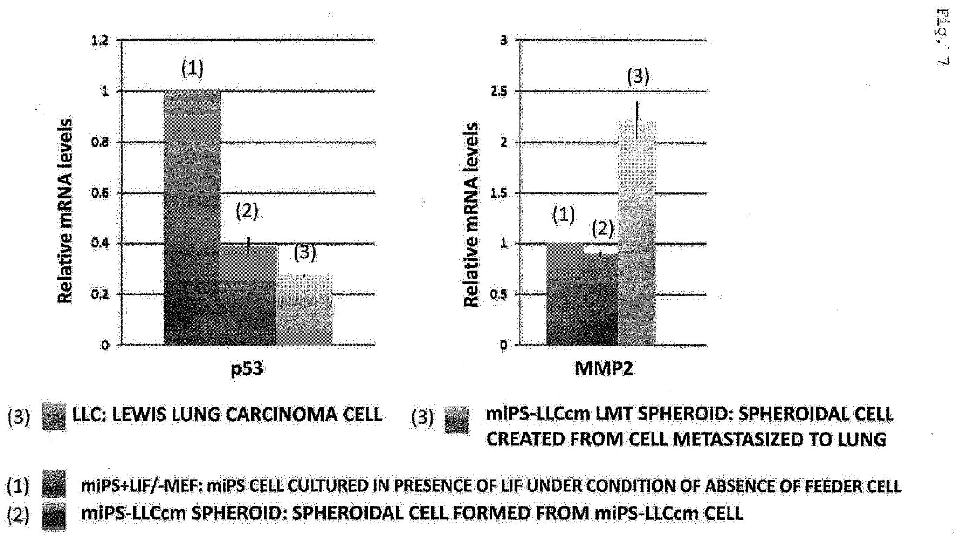

Lung metastasis was caused by injecting the cell population of the present invention in the caudal vein of a nude mouse. Compared to miPS cells, the expression of p53, which is a cancer suppressor gene, decreased in the spheroidal cells formed from miPS-LLCcm cells (FIG. 7, left). Also, the cancer stem cell line miPS-LLCcm LMT, which was established by primary culture of the tumor formed in the lung, showed a remarkably high level of matrix metalloproteinase 2 (MMP2) expression, which is involved in infiltration and metastasis of cancer (FIG. 7, right). These results suggest that cancer stem cells expressing a high level of MMP2 were present in miPS-LLCcm cells, and they preferentially infiltrated into the lung and formed tumor.

### (10) Expression of stem cell markers

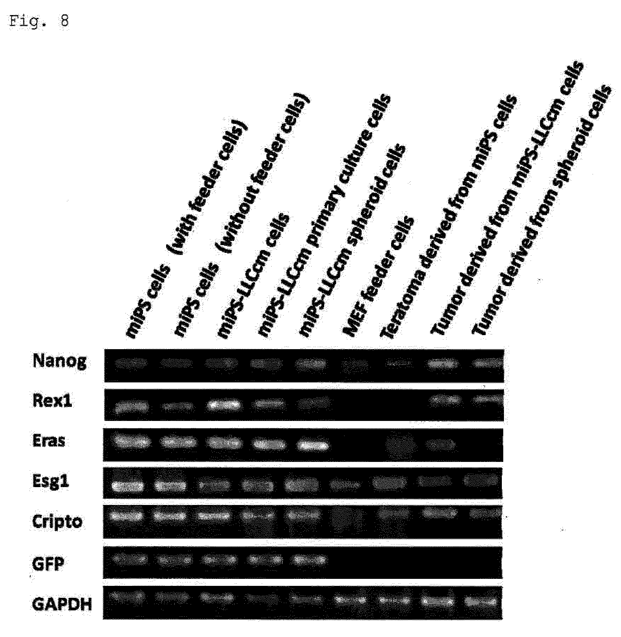

The expression of the stem cell marker gene was examined by RT-PCR in miPS cells (cultured with and without feeder cells), miPS-LLCcm cells, miPS-LLCcm primary cells, and miPS-LLCcm spheroids as well as teratoma and malignant tumor formed by these cells. The RNeasy kit (50) (QIAGEN GmbH) (purified from cells) and TRIzol (Invitrogen Corporation) (purified from tissues) were used. As to the stemness marker genes, those that are presented in a report made by Takahashi and Yamanaka (Cell 126, 663 to 676, 2006, incorporated herein by reference) were examined. As a control, the expression in MEF cells, which were used as feeder cells, was examined. As a result, it was confirmed that the stemness marker genes were expressed in the cell groups obtained by the present invention, and that many of the genes showed an increased expression in tumor samples compared to teratoma (FIG. 8).

### (11) Gene expression analysis by microarray

RNA was extracted from the cell population obtained in this experiment by using the RNeasy kit (50) (QIAGEN GmbH) or using TRIzol (Invitrogen Corporation). Subsequently, 50 µg or less of RNA was treated with DNase I and purified again by the RNeasy kit (50) (QIAGEN GmbH).

From 7.5 µg of RNA, cDNA was synthesized by reverse transcription using SuperScriptII (Invitrogen Corporation) in the presence of 2 mM oligo dT, 1 mM dATP, 1 mM dCTP, 1 mM dGTP, 0.2 mM dTTP, and 0.8 mM aminoalkylated dUTP. After synthesized, cDNA was treated with RNase H. The cDNA thus synthesized was collected by ethanol precipitation and dissolved in 6.7 µl of 0.1 M NaHCO₃ (pH 8.0). An equal amount (6.7 µl) of Cy3 dye was added to the solution, and reacted at 25°C for 120 minutes in the dark. The Cy3-labeled cDNA was purified by the QIAquick column (QIAGEN GmbH). The Cy3-labeled cDNA was hybridized with the mouse cell surface protein gene array slide prepared by the present inventors at 55°C for 15 hours. Subsequently, the fluorescence intensity was detected and analyzed by the FLA8000 scanner (Fujifilm Corporation) and GenePix (R) Pro 5.1 software (Axon instrument).

From the Cy3 fluorescence intensity detected by microarray, the expression levels of the genes in each cell expressed as relative values in relation to those in miPS cells were clustered on a spherical self-organizing map, and genes exhibiting a large difference compared to miPS cells were visualized (FIG. 9). As a result, it was found that there were several patterns of the phenotype of the mapped cell population containing the cancer stem cells generated (FIG. 9). These results suggest that even when the same iPS cells are used, cancer stem cells of different functions and properties are generated depending on the conditions applied.

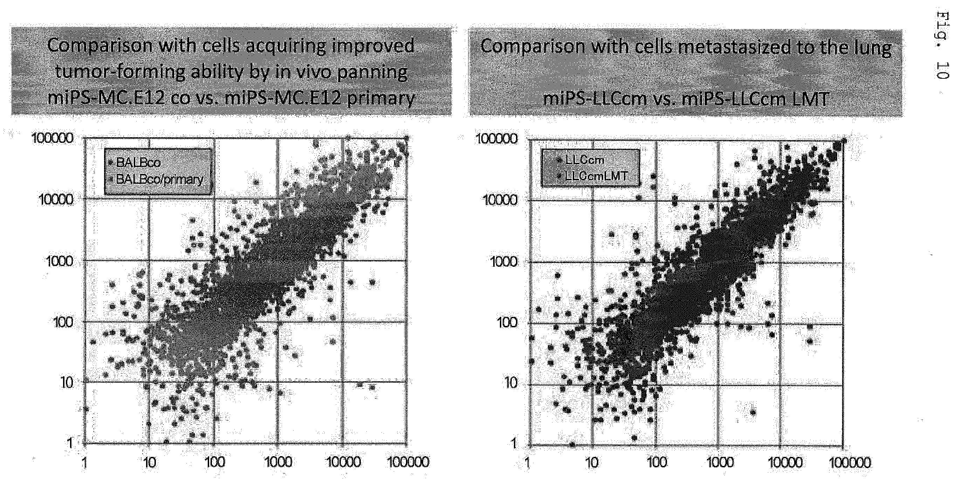

For comparison among individual cells, a scatter plot was created by normalizing the Cy3 fluorescence intensity detected by microarray and plotting the log of the value of the cell to be compared, and gene expression patterns were compared (FIG. 10). Variation in gene expression was also observed between before and after the formation of tumor by miPS-MC.E12co (a cell population obtained by co-culturing miPS cells with BALB MC.E12 cells) (FIG. 10, left) and between miPS-LLCcm and miPS-LLCcm LMT (lung metastatic miPS-LLCcm cells: presumed to be iPS-CSC with high metastatic potential) (FIG. 10, right).

### Example 2. Induction of cancer stem cells from human iPS cells

### (1) Preparation of the culture supernatant of cancer cells (conditioned medium)

The cancer cell lines listed in Table 2 were cultured in adherent culture dishes (100 mm in diameter, manufactured by TPP techno plastic products AG) with the DMEM medium or RPMI1460 medium containing 10% FBS and 1% penicillin/streptomycin under the conditions of 37°C and 5% CO₂.

**[Table 2-1]**

| Cell name (tentative) | Cells used for making CSC | Origin | CM/Exosome/ Other |
|---|---|---|---|
| OCC-hiPS-1 | A172 | Glioma | CM |
| OCC-hiPS-2 | U251-MG | Glioma | CM |
| OCC-hiPS-3 | MDA-MB-415 | Breast | CM |
| OCC-hiPS-4 | MDA-MB-231 | Breast | CM |
| OCC-hiPS-5 | T-47D | Breast | CM |

**[Table 2-2]**

| Cell name (tentative) | Cells used for making CSC | Origin | CM/Exosome/ Other |
|---|---|---|---|
| OCC-hiPS-6 | ZR-75-1 | Breast | CM |
| OCC-hiPS-7 | SK-BR-3 | Breast | CM |
| OCC-hiPS-8 | BT-549 | Breast | CM |
| OCC-hiPS-9 | MFC7 | Breast | CM |
| OCC-hiPS-10 | HT-29 | Colon | CM |

**[Table 2-3]**

| Cell name (tentative) | Cells used for making CSC | Origin | CM/Exosome/ Other |
|---|---|---|---|
| OCC-hiPS-11 | PANC | Pancreas | CM |
| OCC-hiPS-12 | SKOV3 | Ovary | CM |
| OCC-hiPS-13 | HepG2 | Hepatocellular | CM |
| OCC-hiPS-14 | Huh-7 | Hepatocellular | CM |
| OCC-hiPS-15 | PLC/PRF-5 | Hepatocellular | CM |

**[Table 2-4]**

| Cell name (tentative) | Cells used for making CSC | Origin | CM/Exosome/ Other |
|---|---|---|---|
| OCC-hiPS-16 | ECC4 | Gastrointestinal | CM |
| OCC-hiPS-17 | CW-2 | Colon | CM |
| OCC-hiPS-18 | PMF-ko14 | Spleen | CM |
| OCC-hiPS-19 | MY | Lymphocyte | CM |
| OCC-hiPS-20 | MOLT4 | T-cell leukemia | CM |

**[Table 2-5]**

| Cell name (tentative) | Cells used for making CSC | Origin | CM/Exosome/ Other |
|---|---|---|---|
| OCC-hiPS-21 | KLM-1 | Pancreas | CM |
| OCC-hiPS-22 | PK8 | Pancreas | CM |
| OCC-hiPS-23 | PK59 | Pancreas | CM |
| OCC-hiPS-24 | A549 | Lung | CM |
| OCC-hiPS-25 | Li-7 (RPM) | Hepatocellular | CM |

**[Table 2-6]**

| Cell name (tentative) | Cells used for making CSC | Origin | CM/Exosome/ Other |
|---|---|---|---|
| OCC-hiPS-26 | NIH: OVCAR-3 (RPMI) | Ovary | CM |
| OCC-hiPS-27 | Lu99B (RPMI) | Lung | CM |
| OCC-hiPS-28 | RERF-LC-KJ (RPMI) | Ovary | CM |
| OCC-hiPS-29 | A172 | Glioma | CM/primary culture (CB.17 SCID) |
| OCC-hiPS-30 | OVK18 | Ovary | CM |

**[Table 2-7]**

| Cell name (tentative) | Cells used for making CSC | Origin | CM/Exosome/ Other |
|---|---|---|---|
| OCC-hiPS-31 | RERF-LC-AI | Lung | CM |
| | DMEM_control | | |
| | RPMI_contolr | | |

The medium was removed from the dish reaching 80% confluency, and the cells were washed once with the DMEM or RPMI1460 medium containing 5% FBS, and then cultured in the same medium. Twenty four hours after the medium exchange, the culture supernatant was collected and centrifuged under the conditions of 300x g, 10 minutes, and 4°C. The culture supernatant was further centrifuged under the conditions of 2000x g, 10 minutes, and 4°C and filtered through a 0.22 µm-pore filter, and the resulting culture supernatant was used as the conditioned medium.

### (2) Culture of human iPS cells without feeder cells

Human iPS cells (201B, RIKEN BioResource Center) (Takahashi, K. et al., Induction of pluripotent stem cells from adult human fibroblasts by defined factors. Cell 131: 861 to 872 (2007), incorporated herein by reference) were seeded in dishes (60 mm in diameter, manufactured by TPP techno plastic products AG) coated with Laminin-5 (manufactured by ReproCELL Inc.) and cultured with the Repro FF2 medium (manufactured by ReproCELL Inc.) containing 5 ng/ml bFGF under the conditions of 37°C and 2% CO₂. Until the cells reached a stable undifferentiated state in the absence of feeder cells, the entire medium was exchanged every day, and the cells were passaged at a frequency of once a week.

### (3) Induction of cancer stem cells

The conditioned medium collected from each cancer cell line was mixed with Repro FF2 or Repro stem (manufactured by ReproCELL Inc.) or bFGF-free human iPS stem cell medium (DMEM-F12 medium containing non-essential amino acid, 200 mM L-glutamine, KSR, and 0.1 M 2-mercaptoethanol/PBS) at a ratio of 1:1 to prepare a differentiation induction medium. Stable human iPS cells in the undifferentiated state were exposed to each induction medium to induce differentiation. During the induction of differentiation, half of the medium was exchanged every day and the cells were passaged once or twice every two weeks. The period of induction of differentiation was at least 28 days, and at most two months. Culture was performed under the conditions of 37°C and 2% CO₂.

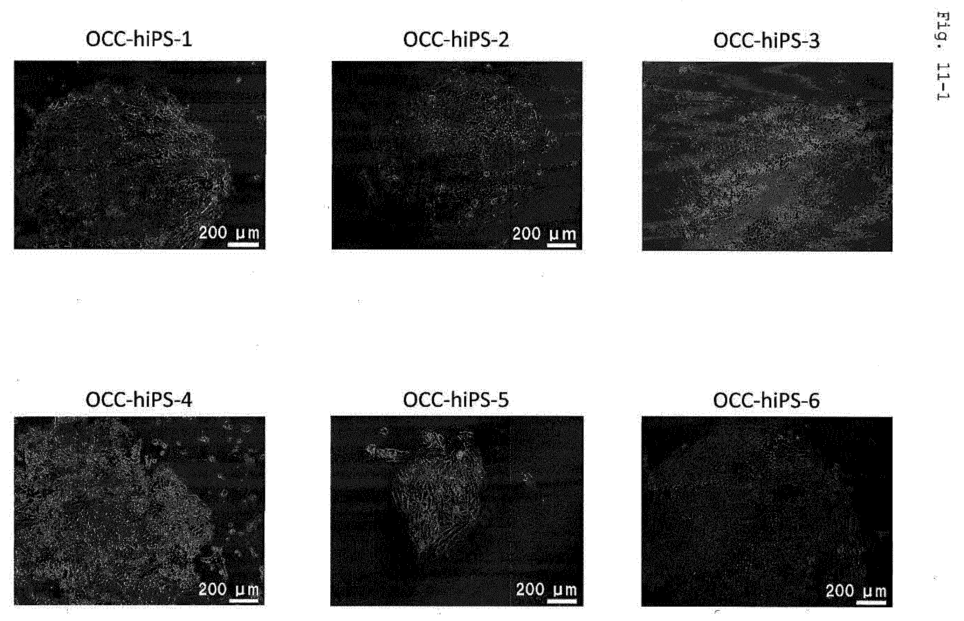

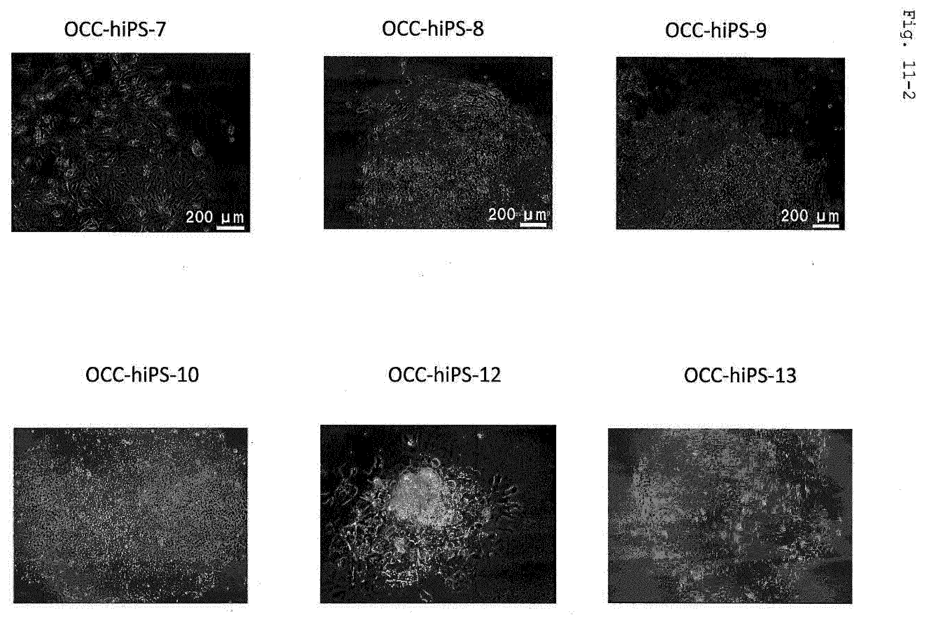

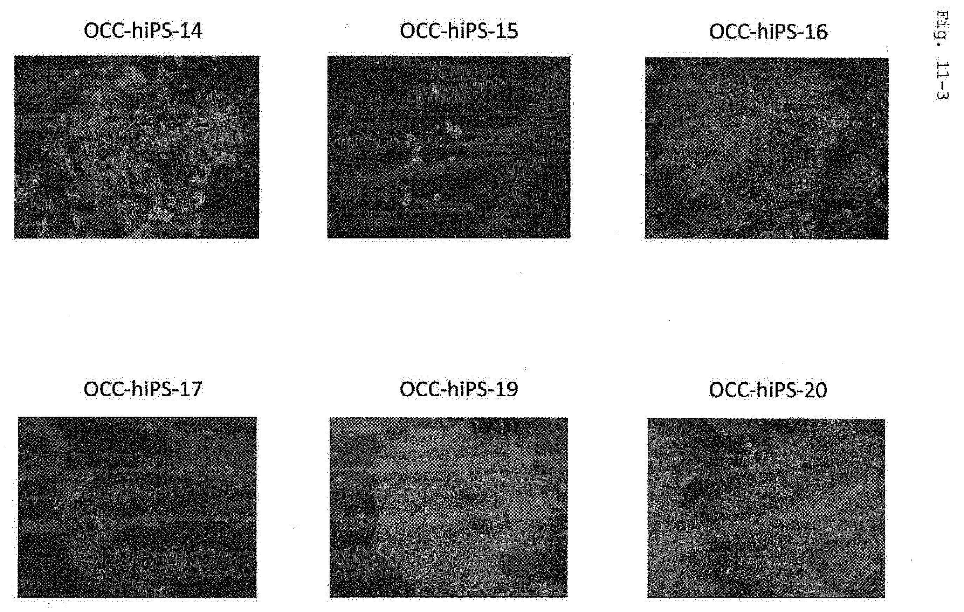

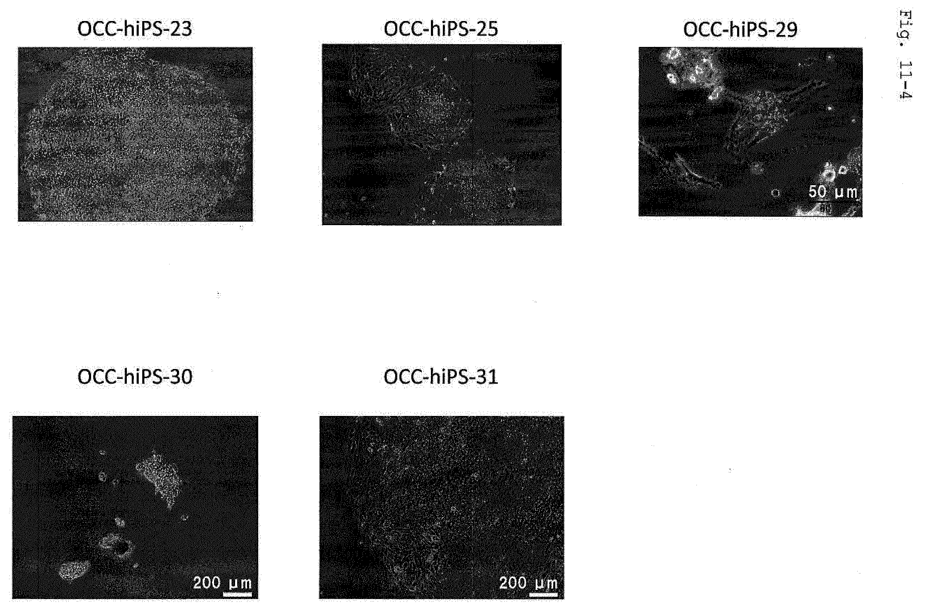

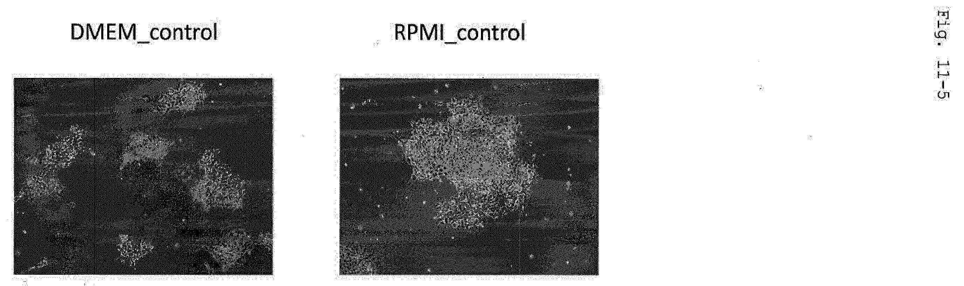

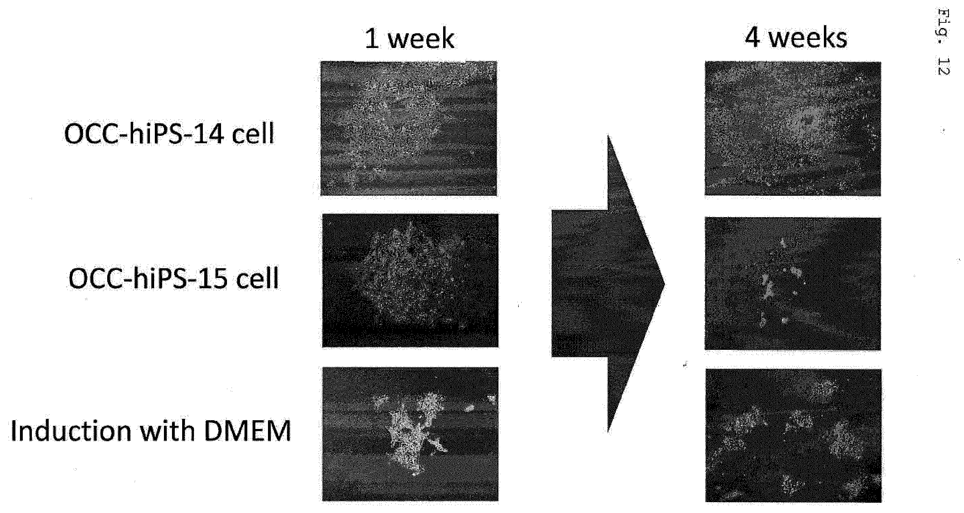

The cells after 28 days of induction of differentiation were observed under the inverted microscope IX81 (Olympus Corporation) and images were captured by MetaMorph using the digital microscope camera DP71 (Olympus Corporation) (FIG. 11-1 to FIG. 11-5). OCC-hiPS-1 continued proliferating while maintaining the stemness even after 28 days of induction, and also, formed tumor in a SCID mouse, as will be described below. Based on the above observation, induction of cancer stem cells from OCC-hiPS-1 was confirmed. Similarly, OCC-hiPS-3, 5, 8, 10, 12, 19, 20, and 23 to 28 continued proliferating while maintaining the stemness even after 28 days of induction. It was revealed that the efficiency of cancer stem cell induction varies depending on the conditioned medium (FIG. 12).

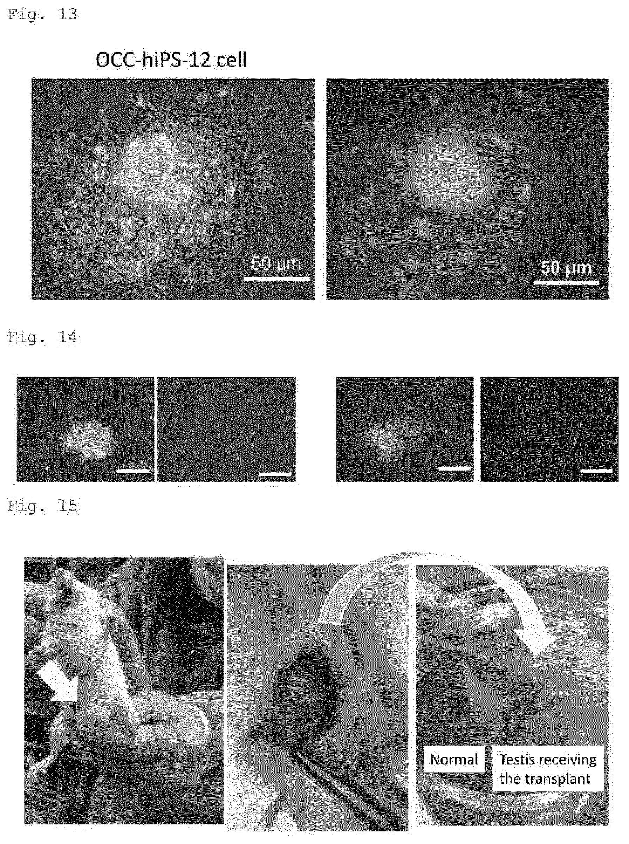

The stemness of a cell line after cancer stem cell induction was examined using rBC2LCN-FITC (manufactured by Wako Pure Chemical Industries, Ltd.). Podocalyxin detected by rBC2LCN-FITC is a glycoprotein ligand specific for human pluripotent stem cells. The detection was carried out in accordance with the protocol of rBC2LCN-FITC. Cells exhibiting green fluorescence by reaction with rBC2LCN were detected (FIG. 13, right), revealing that the cell line after cancer stem cell induction possessed the stemness. Also, when rBC2LCN was not added, green fluorescence was not detected (FIG. 14).

The cell population induced from human iPS cells (OCC-hiPS-1) was suspended in HBSS and transplanted in the testes of Balb/c-nu/nu nude mouse and CB17 scid mouse. Tumor was excised from the CB17 scid mouse into which OCC-hiPS-1 cells were transplanted, where OCC-hiPS-1 cells were confirmed to have formed tumor in the mouse, and the primary culture was established from the tumor (FIG. 15). A part of the tumor was fixed with a 4% paraformaldehyde phosphate buffer solution.

The primary culture cell line (OCC-hiPS-29 cells) was cultured in Laminin-5-coated dishes with the differentiation induction medium prepared using the culture supernatant of A172 cells under the conditions of 37°C and 2% CO₂. The cells were passaged at a frequency of once every three to four days. Also in OCC-hiPS-29 cells, stem cell-like cells reacting with rBC2LCN-FITC were detected

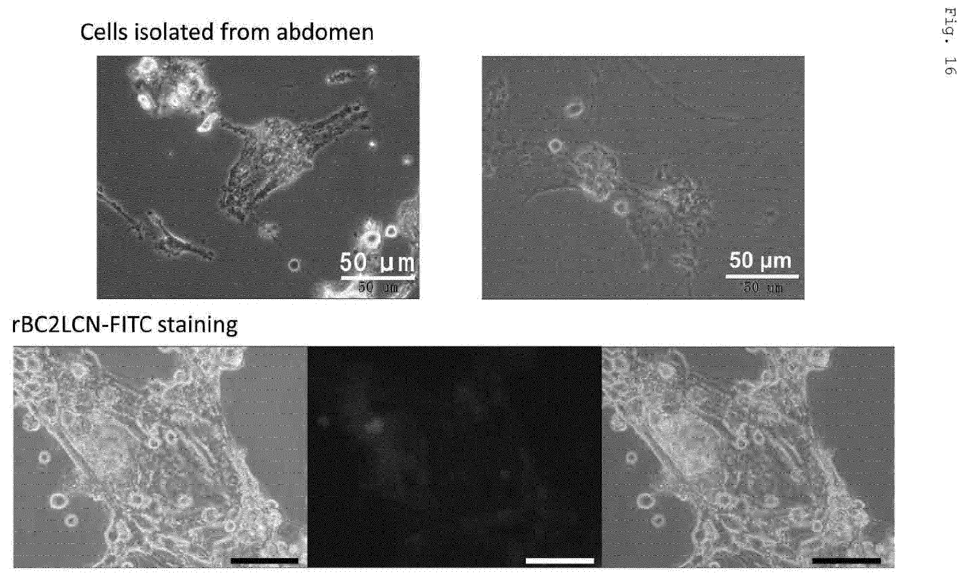

(FIG. 16). The OCC-hiPS-29 cells were cultured in non-adherent dishes with the differentiation induction medium prepared using the culture supernatant of A172 cells for two to five days. A cell-dense region was formed, and in some parts, spheres were formed.

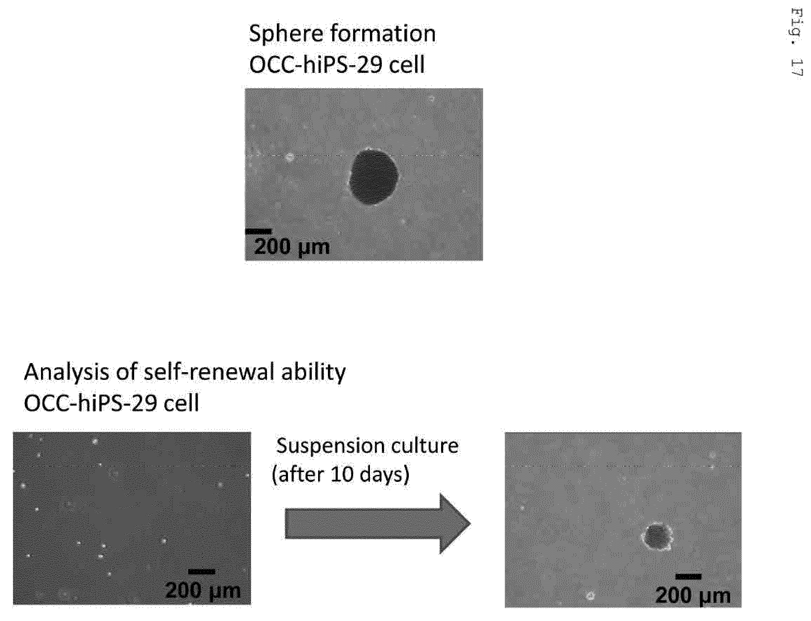

A sphere of the OCC-hiPS-29 cells was treated with the Accutase solution (manufactured by Sigma-Aldrich Co. LLC.) and dispersed in the serum-free medium (DMEM-F12 (manufactured by Sigma-Aldrich Co. LLC.) containing the insulin-transferrin-sodium selenite medium supplement (manufactured by Sigma-Aldrich Co. LLC.), non-essential amino acid (manufactured by Sigma-Aldrich Co. LLC.), and 200 mM L-glutamine (manufactured by Nacalai Tesque, Inc.), and using the same medium, spheroid culture was performed in non-adherent dishes (10 mm in diameter, 10 ml of medium) for 10 days. As a result, spheres were formed, revealing that the cells had the self-renewal ability (FIG. 17).

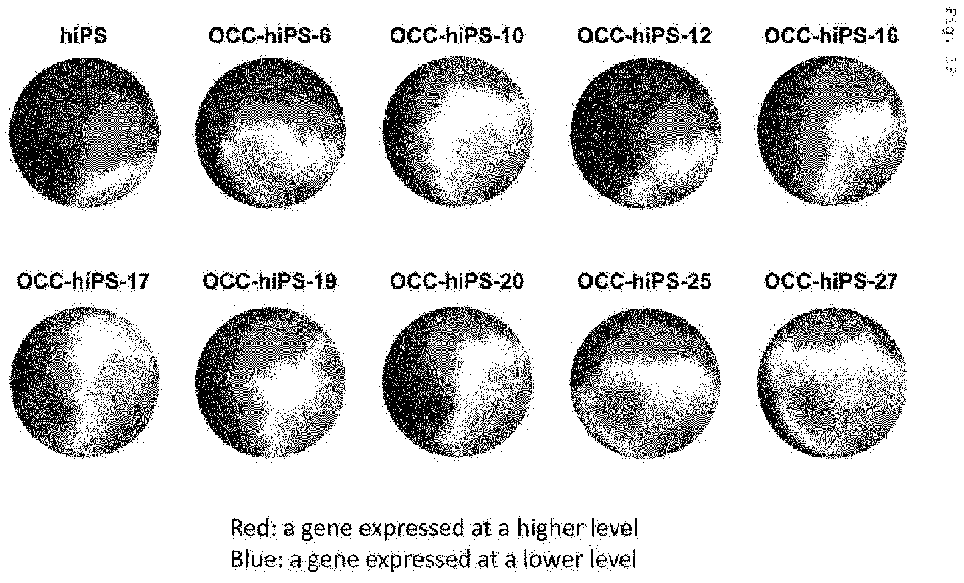

RNA was extracted from the OCC-hiPS-6, OCC-hiPS-10, OCC-hiPS-12, OCC-hiPS-16, OCC-hiPS-17, OCC-hiPS-19, OCC-hiPS-20, OCC-hiPS-25, and OCC-hiPS-27 obtained by the present experiment by a method using the RNeasy kit (50) (QIAGEN GmbH) or a method using TRIzol (Invitrogen Corporation). Microarray was performed by the Agilent Expression Array analysis (one-color method) (analyzed on contract by Takara Bio Inc.). From the Cy3 fluorescence intensity detected by microarray, the expression levels in each cell expressed as relative values were clustered on a spherical self-organizing map to visualize the gene expression levels in each cell. As a result, it was found that there were several patterns of the phenotype of the mapped cell population containing the cancer stem cells generated (FIG. 18). These results suggest that, as with mice, even when the same iPS cells are used, cancer stem cells of different functions and properties are generated depending on the conditions applied.

### Example 3. Concentration of CD44-expressing cells using hyaluronic acid

### (1) Overview

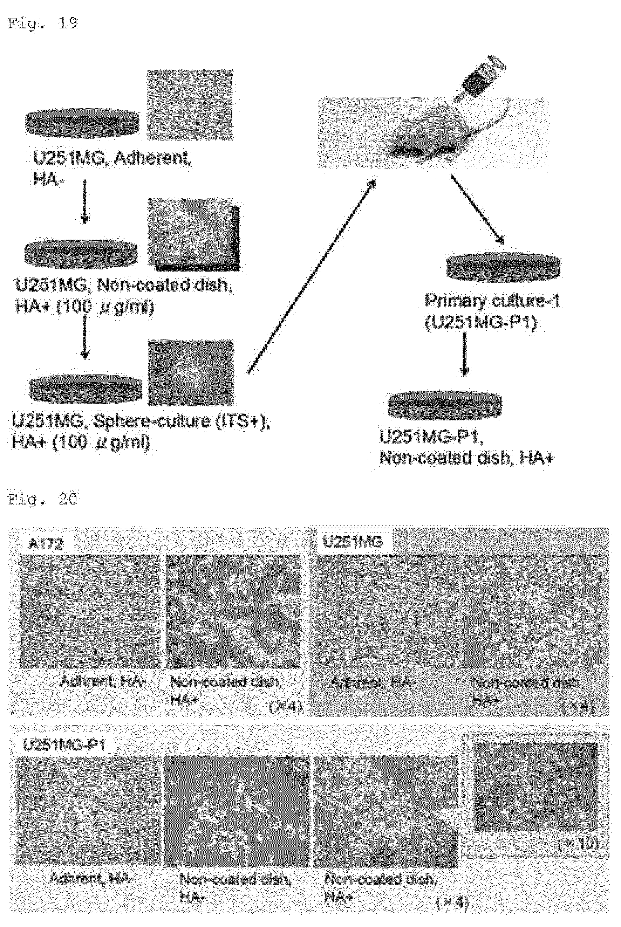

The overview of an experiment using human glioma-derived U251MG cells is illustrated in FIG. 19. At the time of passage, cells which had been cultured in normal adherent dishes were transferred to the medium containing hyaluronic acid (HA) and cultured in non-adherent dishes. The cells were further passaged to form spheres in non-adherent dishes. A nude mouse was inoculated with the spheres to prepare a tumor-bearing mouse. Subsequently, a primary culture (referred to as U251MG-P1) was established in adherent dishes. The primary culture cells were passaged again in the medium with HA and cultured in non-adherent dishes.

### (2) Culture using the medium containing hyaluronic acid

The human glioma-derived A172 cells, U251MG cells, and U251MG-P1 cells were each cultured in non-adherent dishes with the medium with HA (DMEM containing 100 µg/ml sodium hyaluronate (sodium hyaluronate manufactured by Wako Pure Chemical Industries, Ltd., made from chicken combs), 10% FBS, 50 U/ml penicillin, and 50 µg/ml streptomycin) or normal medium without HA (DMEM containing 10% FBS, 50 U/ml penicillin, and 50 µg/ml streptomycin) for two to five days. Cells proliferation was hardly observed in the absence of HA (only the results of U251MG-P1 are shown in FIG. 20), whereas a cell-dense region was formed, and spheres were formed in some parts in the presence of HA (FIG. 20, on the right in each picture). Further, given that proliferation of these cells in normal adherent dishes was confirmed, the cells used were assumed to be in a good condition (FIG. 20, on the left in each picture).

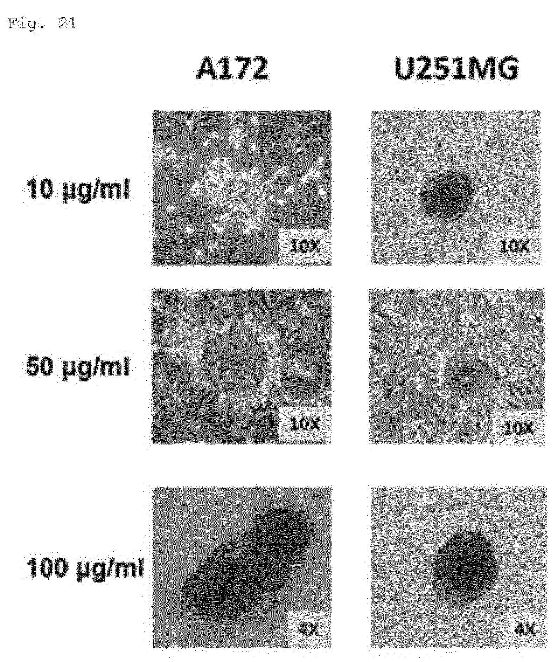

The A172 cells and U251MG cells were each cultured in non-adherent dishes with the medium with HA (DMEM containing 0 to 100 µg/ml sodium hyaluronate, 10% FBS, 50 U/ml penicillin, and 50 µg/ml streptomycin) for five days (FIG. 21). Although the formation of spheres was confirmed at each concentration, spheres were uniformly observed at a concentration of 100 µg/ml. Since no difference was observed between concentrations of 100 µg/ml and 200 µg/ml, HA was added at a concentration of 100 µg/ml in the subsequent assays.

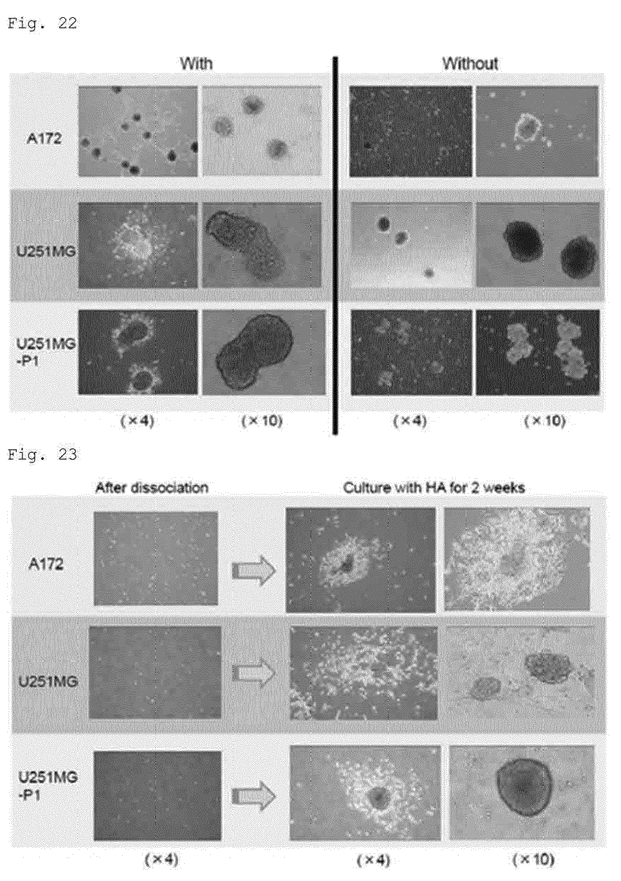

Further, as a result of culture in non-adherent dishes for two weeks in a similar manner, both cell types formed spheres with the addition of HA, and further, cells adhering to the dish surface were observed around the spheres (FIG. 22, With). Although the formation of spheres was observed without the addition of HA, the number of spheres was less than that observed with the addition of HA. Further, once the spheres grew to approximately 200 µm in diameter, they were observed to disintegrate or stop growing, and this was notable in the A172 cells (FIG. 22, Without).

### (3) Confirmation of the sphere-forming ability

The spheres of the A172 cells, U251MG cells, and U251MG-P1 cells prepared in the medium with HA were suspended in Accutase solutions (manufactured by Sigma-Aldrich Corporation) to disperse cells. Subsequently, the cells were each newly cultured in non-adherent dishes with the medium with HA for two weeks (FIG. 23). All cells formed spheres again, and cells adhering to the dish surface were observed around the spheres, showing that the spheres had the self-renewal ability.

### (4) Comparison of the CD44 gene expression level

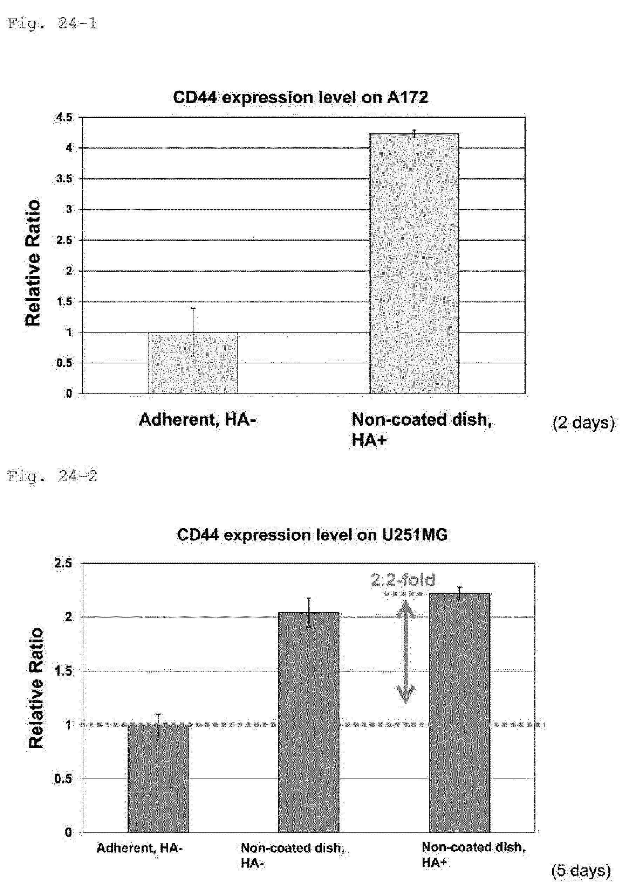

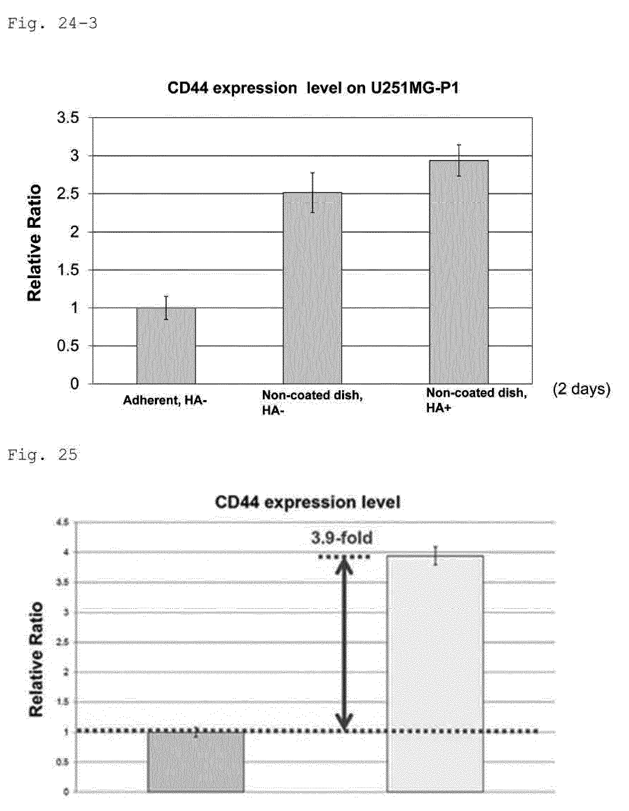

RNA was extracted from the A172 cells, U251MG cells, and U251MG-P1 cells that were cultured in adherent dishes with normal medium and in non-adherent dishes with the medium with HA, respectively, and the CD44 gene expression level was analyzed by qPCR (FIG. 24-1 to FIG. 24-3). With respect to the U251MG cells and U251MG-P1 cells, RNA extraction and comparison were also performed on the cells that were cultured in non-adherent dishes with normal medium.

Compared to normal culture in adherent dishes, the CD44 gene expression was promoted by normal culture in non-adherent dishes. The expression level was even more increased by culture in non-adherent dishes with the medium with HA. In the U251MG cells, the gene expression was approximately 2.2 times higher than that observed in culture in adherent dishes with normal medium.

The U251MG cells and U251MG-P1 cells were cultured in adherent dishes with normal medium, respectively, and the CD44 gene expression level was compared between them (FIG. 25). The expression level in U251MG-P1 was found to be approximately 3.9 times higher than that in U251MG. These results revealed that the CD44 gene expression could be induced or cells expressing a high level of the CD44 gene could be selectively grown by the present culture method, i.e., by spheroid culture of U251MG cells in non-adherent dishes with the medium with HA. Further, in U251MG-P2 cells, cells obtained by primary culture of cancer excised from a nude mouse inoculated with U251MG-P1 cells, the CD44 gene expression level was constantly higher than that in the parent strain cells (data not shown).

### (5) Tumor-forming ability of the U251MG cells after spheroid culture (ITS+, FBS-) in the medium with HA

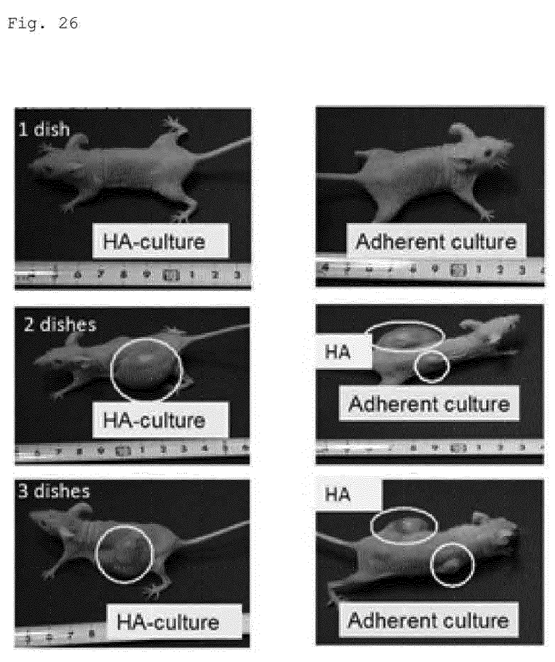

The U251MG cells were cultured in the medium with HA for five days, and then spheroid culture was performed for two weeks in non-adherent dishes (10 mm in diameter, 10 ml of medium) (culture in the medium containing 100 µg/ml sodium hyaluronate and ITS (manufactured by Sigma-Aldrich Corporation)). The resulting cells were transplanted in nude mice, Balb/c-nu/nu, female, 4-week-old, and the mice were observed for four weeks (FIG. 26). The cells collected from one to three dishes were transplanted in the left abdomen of each mouse. In the right abdomen of each mouse, 10⁷ normally cultured U251MG cells were transplanted. Cancer-bearing mice were created by transplantation of cells collected from two or more dishes. The tumor volume ((tumor width² × tumor length)/2) 40 days after transplantation of 10⁷ cells was 1642.8 mm³ (n = 2). Based on the observation that no tumor was formed even when the same number of normally cultured U251MG cells were transplanted under the same conditions, it was shown that the cell population obtained by the method of the present invention had the tumor-forming ability.

### (6) The CD44 protein expression level in the cells subjected to spheroid culture in the medium with HA

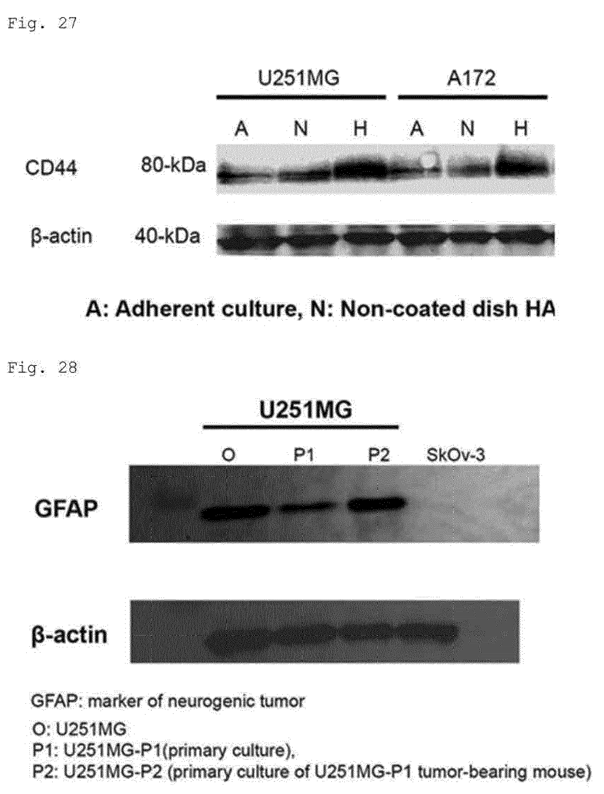

The A172 cells and U251MG cells were cultured in adherent dishes with normal medium (A), non-adherent dishes with normal medium (N), and non-adherent dishes with medium with HA (H), respectively, and the CD44 protein expression level was compared by Western blotting (FIG. 27). Total protein extracted from each of the cells (10 µg) was electrophoresed. As a primary antibody, an anti-CD44 mouse monoclonal antibody (manufactured by Katayama Chemical Industries Co., Ltd.) and an HRP-labeled anti-mouse IgG antibody (manufactured by Cell Signaling Technology Japan, K.K.) were used. Also, using an anti-β-actin antibody (manufactured by Cell Signaling Technology Japan, K.K.) and an HRP-labeled anti-rabbit IgG antibody (manufactured by Cell Signaling Technology Japan, K.K.), β-actin was detected as an internal index of a constitutively expressed protein.

In both cell lines, the expression level of CD44 protein was increased by culture in non-adherent dishes, and the expression level was further increased by culture with the medium with HA.

### (7) Detection of an astrocyte marker protein in U251MG-P1

In order to confirm that the U251MG-P1 cells were derived from glioma, the U251MG cells, U251MG-P1 cells, and U251MG-P2 cells (cells obtained by primary culture of cancer excised from a nude mouse inoculated with the U251MG-P1 cells) were cultured in adherent dishes with normal medium, and GFAP protein, which is a marker protein of astrocyte, was detected by Western blotting (FIG. 28). Also, protein extracted from cells not expressing GFAP (human ovarian cancer-derived SkOv-3 cells) was used as a negative control. Total protein extracted from each of the cells (10 µg) was electrophoresed, and then detected using an anti-GFAP antibody (manufactured by Santa Cruz Biotechnology, Inc.) and an HRP-labeled anti-mouse IgG antibody (manufactured by Cell Signaling Technology Japan, K.K.). As an internal index of a constitutively expressed protein, β-actin was detected.

With respect to each of the above cell lines, the U251MG cells were indicated as 0, U251MG-P1 cells as P1, U251MG-P2 cells as P2, and SkOv-3 cells as SkOv-3 in FIG. 28. The U251MG-P1 and U251MG-P2 cells were confirmed to express GFAP when cultured in adherent dishes.

### (8) HA culture of the SkOv-3 cells

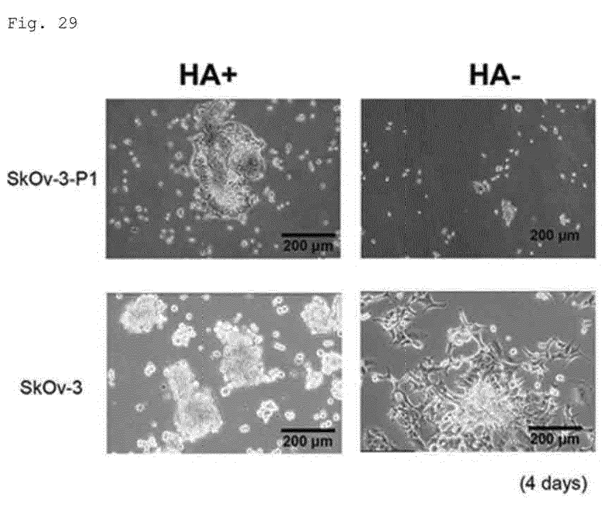

As for the U251MG cells, the SkOv-3 cells and SkOv-3-P1 cells (cells obtained by primary culture of cancer excised from a mouse inoculated with the SkOv-3 cells) were cultured, respectively, in non-adherent dishes with the medium with HA (RPMI1640 containing 100 µg/ml sodium hyaluronate (sodium hyaluronate manufactured by Wako Pure Chemical Industries, Ltd., made from chicken combs), 10% FBS, 50 U/ml penicillin, and 50 µg/ml streptomycin) or in non-adherent dishes with normal medium (RPMI1640 containing 10% FBS, 50 U/ml penicillin, and 50 µg/ml streptomycin) for four days (FIG. 29).

With respect to the SkOv-3 cells, although spheres were observed in the medium with HA, no sphere was observed in the medium without HA (HA-, normal medium). With respect to the SkOv-3-P1 cells, spheres grew in size in the presence of HA, and there were many cells adhering to the dish surface around the spheres. In the medium without HA (normal medium), a small number of cells adhering to the dish surface formed small spheres; however, in comparison with culture with the medium with HA, the growth was slower and the spheres were smaller in size. In adherent dishes, there was almost no difference between the presence and absence of HA, and the spheres grew normally (data not shown).

## Claims

1. A method of obtaining a cell population containing cancer stem cells, which comprises culturing iPS cells in the presence of culture supernatant of cancer cells.

2. The method of Claim 1, wherein the iPS cells are human cells.

3. The method of Claim 1 or 2, wherein the period of culture is 2 weeks to 2 months.

4. The method of any one of Claims 1-3, wherein the cell population obtained by the method contains cancer cells.

5. A cell population obtained by the method of any one of Claims 1-4.

6. A method of obtaining cancer stem cells, which comprises isolating cancer stem cells contained in the cell population of Claim 5.

7. Cancer stem cells obtained by the method of Claim 6.

8. A method of screening an anti-cancer agent, which comprises the steps of:
(1) bringing the cell population containing cancer stem cells of Claim 5 or the cancer stem cells of Claim 7 into contact with a candidate of an anti-cancer agent, and
(2) determining the effect of the candidate on the cell population or the cells.

9. The method of Claim 8, further comprising the step of selecting a candidate which inhibits proliferation of the cell population or the cells.

10. The method of Claim 8 or 9, which comprises the step of bringing the cell population containing cancer stem cells of Claim 5 into contact with a candidate of an anti-cancer agent.

11. A method of concentrating CD44-expressing cells, which comprises culturing a cell population containing CD44-expressing cells in the presence of hyaluronic acid under the non-adherent condition.

12. The method of Claim 11, wherein the cell population is a human cell population.

13. The method of Claim 11 or 12, wherein the period of the culture is 2 weeks to 2 months.

14. A cell population containing CD44-expressing cells concentrated by the method of any one of Claims 11-13.
